# EUROPÄISCHE PATENTSCHRIFT

(11) **EP 2 190 809 B1**
(45) Veröffentlichungstag und Bekanntmachung des Hinweises auf die Patenterteilung: **08.06.2011**
(21) Anmeldenummer: 08802340.3
(22) Anmeldetag: 18.09.2008
(51) Int. Cl.: C07C 67/30, C07C 231/18, C07C 69/24, C07C 69/612, C07C 69/40, C07C 69/708, C07C 233/07, C07B 53/00, C07B 55/00, C07C 51/347, C07C 53/128, C07B 37/04, C07B 49/00, C07C 231/12

(54) **VERFAHREN ZUR HERSTELLUNG OPTISCH AKTIVER ALPHA-ALKYLCARBONYLVERBINDUNGEN**
METHOD FOR THE PRODUCTION OF OPTICALLY ACTIVE ALPHA ALKYL CARBONYL COMPOUNDS
PROCÉDÉ POUR PRODUIRE DES COMPOSÉS -ALKYLCARBONYLE OPTIQUEMENT ACTIFS

(30) Priorität: 24.09.2007 DE 102007045624
(43) Veröffentlichungstag der Anmeldung: 02.06.2010
(73) Patentinhaber: Albert-Ludwigs-Universität Freiburg, 79088 Freiburg (DE)
(72) Erfinder: BREIT, Bernhard, 79194 Gundelfingen (DE); STUDTE, Christopher, 113-0032 TOKYO (JP)
(74) Vertreter: Keller, Günter
(86) Internationale Anmeldenummer: PCT/EP2008/007818
(87) Internationale Veröffentlichungsnummer: WO 2009/040054

(56) Entgegenhaltungen:
- WO-A-2007/013555
- US-A- 4 723 033
- C.STUDTE AND B. BREIT: "Zinc-catalyzed enantiospecific sp3-sp3-cross-coupling of alpha-hydroxy ester triflates with Grignard reagents" ANGEW.CHEM.INT.ED., Bd. 47, 2008, Seiten 5451-5455, XP002513473
- M. HATANO ET AL: "Highly efficient alkylation to ketones and aldimines with Grignard reagents catalyzed by zinc(II) chloride" J.AM.CHEM.SOC., Bd. 128, Nr. 31, 2006, Seiten 9998-9999, XP002513474
- Y. PETIT ET AL: "Stereoselective synthesis of optically active alpha-methyl esters" TETRAHEDRON LETT., Bd. 31, Nr. 15, 1990, Seiten 2149-2152, XP002513475 in der Anmeldung erwähnt
- S. HANESSIAN ET AL: "Heteroatom-assisted substitution of acyclic secondary tosylates with lithium dialkylcuprates. An expedient route to stereochemically defined deoxypropionate and related biosynthetic subunits" J.ORG.CHEM., Bd. 54, Nr. 25, 1989, Seiten 5831-5833, XP002513476

## Beschreibung

Die vorliegende Erfindung betrifft ein Verfahren zur Herstellung von optisch aktiven α-Alkylcarbonylverbindungen unter Erhalt der Stereoinformation der Ausgangsverbindungen. Hierbei werden als Ausgangsverbindungen Carbonylverbindungen eingesetzt, die in α-Stellung zur Carbonylgruppe eine Abgangsgruppe aufweisen, die unter Inversion der Konfiguration durch eine Alkyl-, Alkenyl- oder Arylgruppe substituiert wird. Die Substitution der Abgangsgruppe erfolgt unter Verwendung eines Alkyl-, Alkenyl- oder Arylmagnesium-Grignards und eines Zink(II)salzes oder eines Zinkorganyls.

Optisch aktive α-Alkylcarbonylverbindungen sind wichtige Intermediate in der Synthese zahlreicher medizinischer Wirkstoffe. Für ihre Herstellung gibt es eine Vielzahl unterschiedlicher Möglichkeiten. Entscheidend ist hierbei, dass das Herstellungsverfahren einen sehr hohen Enantiomerenüberschuss (*ee*) des Produkts ermöglicht, damit dieses für die Wirkstoffsynthese verwendet werden kann.

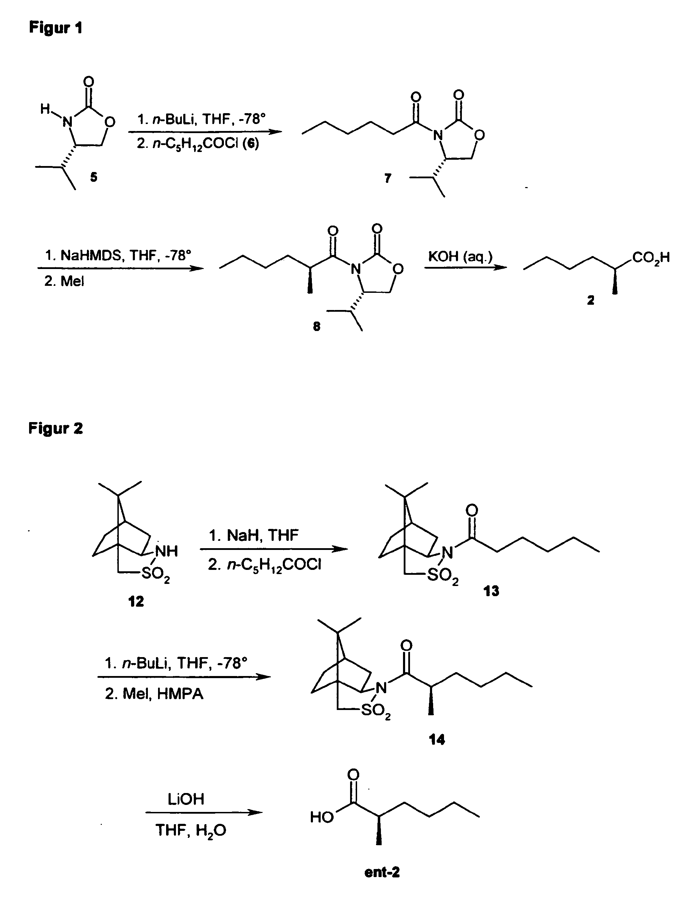

Eine Möglichkeit zur Darstellung der optisch aktiven α-Alkylcarbonylverbindungen ist die α-Alkylierung chiraler Enolate (Evans et al., Asymmetric synthesis, 1984, Morrison J.D., Ed.; Academic Press, New York, Vol. 3, p. 1; Oppolzer et al., Helv. Chim. Acta, 1985, 68, 212). Die von Evans et al. entwickelte Oxazolidinon-Methode verwendet hierzu ein Oxazolidinon als Hilfsstoff, an den zunächst die Carbonylverbindung, die alkyliert werden soll, addiert wird. Anschließend wird an diese Zwischenstufe in der Enolatform eine Alkylgruppe addiert (siehe Figur 1, sowie Evans et al., J. Am. Chem. Soc., 1981, 103, 2127).

Bei dem Verfahren von Oppolzer et al. wird ein Sultam als Hilfsstoff verwendet (siehe Figur 2), das nach α-Alkylierung zu einer chiralen α-Alkylcarbonsäure hydrolysiert werden kann (Oppolzer et al., Tetrahedron Lett., 1989, 30, 5603).

Obwohl die oben beschriebenen Verfahren eine hohe Enantioselektivität ermöglichen, eignen sich die chiralen Auxiliare nicht gut für die Synthese im großen Maßstab. Insbesondere müssen die chiralen Auxiliare oftmals für die Ausgangsverbindung optimiert werden, wodurch ein derartiges Verfahren besonders umständlich wird. Andere Verfahren gehen von chiralen α-Chlorketonen (Ready et al., J. Am. Chem. Soc., 2004, 126, 10240) oder racemischen α-Bromamiden unter Verwendung von chiralen Liganden aus (Fu et al., J. Am. Chem. Soc., 2005, 127, 4594). Hierbei treten ebenfalls erhebliche Nachteile auf, da die Verfahren meist Substrat-spezifisch sind und/oder teure und teils toxische Katalysatoren benötigen. Des Weiteren kann oftmals nicht die gewünschte Enantiomerenreinheit erreicht werden bzw. kommt es manchmal zum kompletten Verlust der Stereoinformation, wie im Fall der kostengünstigen Eisenkatalysatoren (Cahiez et al., Angew. Chem., 2007, 119, 1).

Ein anderer Syntheseansatz ist die Racemattrennung bzw. Racematspaltung, die beispielsweise unter Verwendung eines Chininsalzes durchgeführt werden kann (Levene et al., J. Biol. Chem., 1926, 70, 211). Hierbei wird zunächst 2-Methylhexansäure in das Chininsalz überführt und durch Umkristallisation in die Racemate aufgetrennt. Obwohl das Verfahren der Racemattrennung kostengünstig ist, kommt es durch die Umkristallisation zu hohen Ausbeuteverlusten.

Ein weiteres Verfahren ist die Esterhydrolyse unter Verwendung von Enzymen, wobei beispielsweise Ester der racemischen 2-Methylhexansäure unter Verwendung von Lipasen und Esterasen hydrolysiert werden (Ozaki et al., Chem. Abs., 1997, 127, 276440). Diese Verfahren sind jedoch ebenfalls nicht gut für die großtechnische Anwendung geeignet, da die benötigten Enzyme sehr teuer sind und, obwohl die Enzyme teils einen hohen Enantiomerenüberschuss liefern, die Reaktion langsam verläuft und nur eine geringe Konversionsrate ermöglicht.

Die Herstellung von optisch aktiven α-Alkylcarbonylverbindungen kann auch durch asymmetrische Hydrierung erfolgen. Besonders geeignet ist hierbei der Ruthenium-BINAP-Komplex (Asymmetric Synthesis, Morrison J.D., Ed; Academic Press, 1985, Vol. 5). Dieses Verfahren ist zwar für einen großtechnischen Maßstab geeignet, jedoch erfordert es eine aufwendige und komplizierte Technik und die Katalysatoren sind meist Substrat-spezifisch. Im Falle der 2-Methylhexansäure liefert sie letztlich nur einen Enantiomerenüberschuss von 90 %. Zudem ermöglicht auch die asymmetrische Hydrierung kein kostengünstiges Herstellungsverfahren.

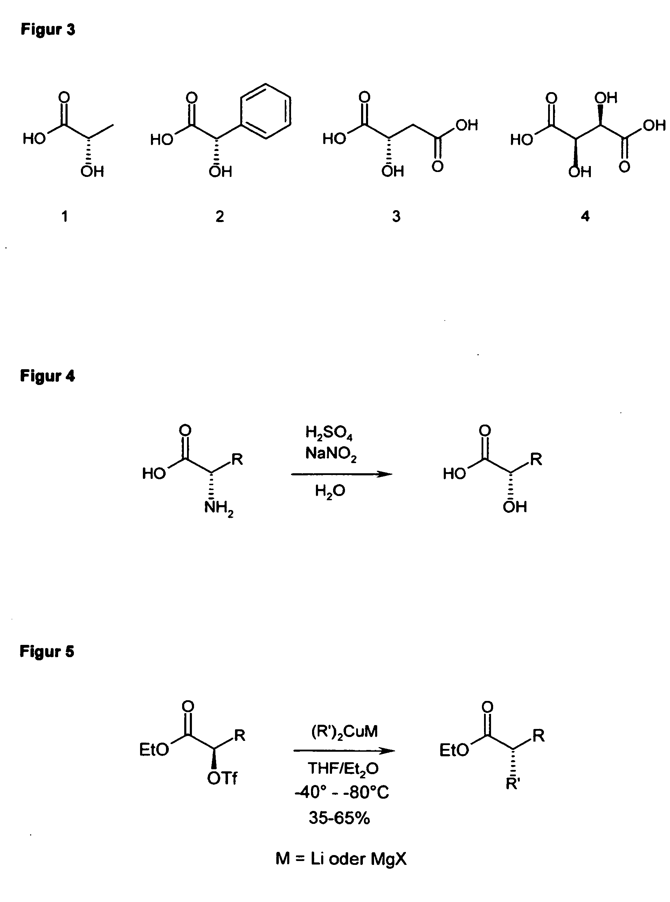

wesentlich einfacher als die stereoselektive Synthese ist der Zugang zu enantiomerenreinen α-Hydroxyestern, die aus dem *chiral pool* (natürlich vorkommende enantiomerenreine Verbindungen) gewonnen werden können (Figur 3). Hierzu zählen die natürlich vorkommenden α-chiralen Aminosäuren, die sich leicht, unter Erhalt der Stereoinformation, in die entsprechenden α-Hydroxysäuren überführen lassen, wodurch die Substratpalette um eine weitere sehr wichtige Substanzklasse erweitert wird (Figur 4).

Larchevêque et al. konnten zeigen, dass sich α-Trifluorsulfoxyester stereoselektiv mit stöchiometrisch eingesetzten Dialkylcupraten substituieren lassen. Aufgrund konkurrierender Eliminierungs- und Reduktionsreaktionen liegen die hierbei erzielten Ausbeuten mit verschiedenen Grignard-Reagenzien jedoch nur bei 35-65 % (Figur 5). Außerdem erfordert die Reaktionsführung mindestens 2 Äquivalente des Alkylierungsreagenzes und eine stöchiometrische Menge an Kupfersalz, sowie tiefe Temperaturen von bis zu -80°C (Larchevêque et al., Tet. Lett., 1990, 31, 2149).

Hatano et al., J.Am.Chem.Soc. (2006), 128, SS 9998-9999 offenbaren die Verwendung von Grignard-Reagenzien und Zink(II)chlorid zur Alkylierung von Ketonen und Aldiminen, wobei Alkyl-substituierte Alkohole bzw. Amine hergestellt werden.

Petit et al., Tetrahedron Letters, Vol. 31 (1990), SS 2149-2152 offenbaren die stereoselektive Synthese von optisch aktiven α-Methylestern ausgehend von den entsprechenden Triflat-aktivierten Estern. Als Katalysator verwendet wurden R₂CuMgX bzw. (CH₃)₂CuLi.

Hanessian et al., J.Org.Chem. (1989), SS 5813-5833 offenbaren die stereoselektive Synthese optisch aktiver Alkohole und Thioether. Dabei reagieren Tosylat-aktivierte Verbindungen mit Lithiumdialkylcupraten.

Eine Aufgabe der vorliegenden Erfindung ist es daher, ein Verfahren zur Herstellung von chiralen α-Alkylcarbonylverbindungen mit hohem Enantiomerenüberschuss zur Verfügung zu stellen, das auf einfache Weise, unter Verwendung einer katalytischen Menge an kostengünstigem und ungiftigem Metallsalz, sowie leicht handhabbaren und einfach darzustellenden Ausgangsverbindungen durchgeführt werden kann. Eine weitere Aufgabe ist es hierbei, Reaktionsbedingungen zu finden, die eine Tieftemperaturkühlung unnötig machen.

Diese Aufgaben werden durch das erfindungsgemäße Verfahren, wie in den Ansprüchen beschrieben, gelöst. Die vorliegende Erfindung betrifft daher ein Verfahren zur Herstellung von Verbindungen der Formel I: 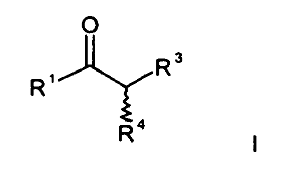 worin eine Verbindung der Formel II, unter Inversion der Stereokonfiguration am α-Carbonylkohlenstoffatom, 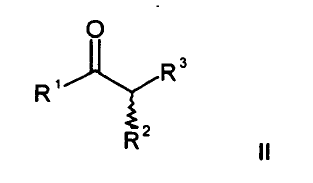 mit R⁴MgX und ZnY₂ oder R⁴MgX und ZnR⁴₂, bevorzugt mit R⁴MgX und ZnY₂, zu einer Verbindung gemäß Formel I umgesetzt wird,
worin R¹ gleich OM, O-R⁵ oder NR⁵R⁶ sein kann, wobei M ein Metallion, bevorzugterweise Natrium oder Kalium, und R⁵ und R⁶ unabhängig voneinander einen geradkettigen oder verzweigten Alkylrest, Alkenylrest Arylalkylrest, Arylrest oder Heteroarylrest darstellen können,
worin R² eine Sulfonatabgangsgruppe, eine Phosphatabgangsgruppe, eine Carboxylatabgangsgruppe, eine Carbamatabgangsgruppe, eine Carbonatabgangsgruppe oder ein Halogenid ist,
worin R³ einen geradkettigen oder verzweigten Alkylrest, Alkenylrest, Arylalkylrest, Arylrest oder Heteroarylrest darstellt, der 5-8 Kohlenstoffatome, sowie 1-2 Heteroatome, ausgewählt aus O N, P und S, als Substituenten aufweisen oder von den Heteroatomen unterbrochen werden kann,
worin R⁴ einen geradkettigen oder verzweigten Alkylrest, Alkenylrest, Arylalkylrest, Arylrest oder Heteroarylrest darstellt,
worin bei R¹ bis R⁶ ein Alkylrest 1-15 Kohlenstoffatome, ein Alkenylrest 2-15 Kohlenstoffatome, ein Arylalkylrest 5-15 Kohlenstoffatome, ein Arylrest 5-10 Kohlenstoffatome und ein Heteroarylrest 5-8 Kohlenstoffatome, sowie 1-2 Heteroatome, ausgewählt aus O N, P und S, aufweisen kann,
worin X ein Halogen, bevorzugt Chlor, ist,
und worin Y ein Sulfonat, ein Sulfat, ein Carboxylat, ein Alkoholat, ein Nitrat, ein Chalkogen oder ein Halogen, bevorzugterweise ein Halogen, weiter bevorzugt Chlor, darstellen kann.
Weiter bevorzugt wird das erfindungsgemäße Verfahren bei Temperaturen von mehr als -30°C durchgeführt.

Noch weiter bevorzugt werden in dem erfindungsgemäßen Verfahren die Verbindungen der Formel II mit einem Enantiomerenüberschuss (*ee*) von mehr als 95 % eingesetzt und die Verbindungen der Formel I mit einem Enantiomerenüberschuss von mehr als 95 % hergestellt.

In einer weiter bevorzugten Ausführungsform des erfindungsgemäßen Verfahrens werden 0,5 - 25 Gew.-% ZnY₂ oder ZnR⁴₂, bezogen auf die Menge der Verbindung der Formel II, verwendet.

Weiter bevorzugt wird in dem erfindungsgemäßen Verfahren als Lösungsmittel für die Reaktion Tetrahydrofuran, 2-Methyltetrahydrofuran oder Diethylether bzw. ein Gemisch aus diesen Lösungsmitteln verwendet.

In einer weiter bevorzugten Ausführungsform des erfindungsgemäßen Verfahrens sind die Reste R¹ bis R⁶ dadurch gekennzeichnet, dass ein Alkylrest 1-10 Kohlenstoffatome, ein Alkenylrest 2-10 Kohlenstoffatome, ein Arylalkylrest 5-10 Kohlenstoffatome, ein Arylrest 5-8 Kohlenstoffatome und ein Heteroarylrest 5-7 Kohlenstoffatome, sowie 1-2 Heteroatome, ausgewählt aus O, N, P und S, umfasst.

Bei dem Rest R² handelt es sich um eine Alkylsulfonsäuregruppe, eine Phosphat-, Carboxylat-, Carbamat- oder Carbonatgruppe oder um ein Halogen.

In einer bevorzugten Ausführungsform des erfindungsgemäßen Verfahrens stellt R² eine Alkylsulfonsäuregruppe, weiter bevorzugt eine fluorierte Alkylsulfonsäuregruppe mit 1-9 Fluoratomen, noch weiter bevorzugt eine Trifluormethansulfoxygruppe oder eine Perfluorbutansulfoxygruppe dar.

Noch weiter bevorzugt stellt R⁴ eine Methyl-, Ethyl-, *i*Propyl-, *n*Butyl-, *i*Butyl-, *s*Butyl-, Cyclohexyl-, Octyl-, Lauryl-, Allyl-, Metallyl-, Vinyl-, Phenyl-, Benzyl-, 3-Benzyloxypropyl- und 3-Benzyloxy-2-methylpropylgruppe dar.

In einer weiter bevorzugten Ausführungsform des erfindungsgemäßen Verfahrens werden 1-2 Äquivalente R⁴MgX , bezogen auf die Stoffmenge der Verbindung der Formel II, verwendet.

In einer noch weiter bevorzugten Ausführungsform des erfindungsgemäßen Verfahrens werden die Verbindungen der Formel II aus natürlich vorkommenden, chiralen Verbindungen hergestellt.

In einer anderen bevorzugten Ausführungsform des erfindungsgemäßen Verfahrens werden die Verbindungen der Formel II hergestellt aus der Gruppe bestehend aus: Aminosäuren, L-Milchsäure, L-Äpfelsäure, L-Mandelsäure, L-Weinsäure.

Die "geschlängelte" Darstellung der C-C-Bindung zwischen R⁴ und dem α-Kohlenstoff der Carbonylverbindung sowie R² und dem α-Kohlenstoff der Carbonylverbindung in den Figuren und den Formeln I und II deutet an, dass R⁴ bzw. R² in Bezug auf die Papierebene entweder "nach vorne" oder "nach hinten" stehen kann. Das erfindungsgemäße Verfahren führt zur Inversion der Stereokonfiguration in α-Stellung zur Carbonylgruppe, so dass R⁴ in Formel I "nach vorne steht", wenn R² in der Ausgangsverbindung gemäß Formel II "nach hinten steht". Im anderen Fall steht R⁴ "nach hinten", wenn R² in der Ausgangsverbindung der Formel II "nach vorne steht".

Das erfindungsgemäße Verfahren ermöglicht die Herstellung von α-Alkylcarbonylverbindungen bei relativ hohen Temperaturen (beispielsweise 0°C). Vorteilhafterweise ermöglicht das erfindungsgemäße Verfahren die Verwendung von kostengünstigem, nicht-toxischem, umweltverträglichem ZnCl₂ in katalytischen bzw. sehr geringen Mengen. Durch das erfindungsgemäße Verfahren und die Verwendung der einfach darzustellenden Organometallreagenzien können verschiedenste Carbonylverbindungen, die in α-Stellung eine Abgangsgruppe aufweisen, in hohen Ausbeuten zu α-Alkylcarbonylverbindungen umgesetzt werden.

Des Weiteren sind die für das erfindungsgemäße Verfahren benötigten OrganometallReagenzien einfach zu handhaben, kostengünstig und finden in der Industrie bereits große Verwendung. Daher ist das erfindungsgemäße Verfahren besonders gut für die Produktion im großen Maßstab geeignet. Vorteilhafterweise lassen sich die optisch aktiven Carbonylverbindungen mit einer Abgangsgruppe in α-Stellung ohne chromatographische Trennmethoden einfach aus den kostengünstigen, natürlich vorkommenden chiralen Verbindungen synthetisieren, die zudem relativ stabil sind und sich bei tiefen Temperaturen über einen langen Zeitraum lagern lassen. Auf die Verwendung von teuren chiralen Liganden oder Auxiliaren kann verzichtet werden. Durch die Reaktionsführung bei relativ hohen Temperaturen kann auf eine teure Tieftemperatur-Kühltechnik verzichtet werden und die Reaktionen können dennoch - gegenüber Reaktionen bei Raumtemperatur- auch in großen Reaktionsreaktoren gut kontrolliert werden. Zudem ist teilweise die Rückgewinnung der Abgangsgruppe möglich, wodurch die Herstellungskosten weiter reduziert werden können. Des Weiteren ist die Aufarbeitung und die Isolierung der Reaktionsprodukte unter Verwendung einer fraktionierten Destillation leicht durchführbar.

Im Gegensatz zur Enolatchemie, bei der die Alkylreste als Elektrophile eingeführt werden, bietet das erfindungsgemäße Verfahren einen effizienten und allgemeinen Zugang zu optisch aktiven α-Alkylcarbonylverbindungen über die Einführung des Alkylrestes als Nukleophil in ausgezeichneten Ausbeuten. Besonders durch den Zugang zu enantiomerenreinen α-Alkylcarbonylverbindungen mit sekundären Resten in der α-Position (siehe Beispiele) bietet das erfindungsgemäße Verfahren einen Vorteil gegenüber der chiralen Enolatchemie, für die sekundäre Elektrophile ungeeignet sind.

Aber auch die von Fu et al. (Fu et al., J. Am. Chem. Soc., 2005, 127, 4594) beschriebene stereoselektive Alkylierung mit Nukleophilen bietet keine effiziente und allgemeine Methode zur selektiven Einführung eines sekundären Restes. Hingegen sind bei dem erfindungsgemäßen Verfahren selbst β-verzweigte Carbonylverbindungen mit einer Abgangsgruppe in der α-Position als Substrate geeignet (siehe Beispiele), wodurch vicinale Stereozentren aufgebaut werden können.

Ein weiterer Vorteil des erfindungsgemäßen Verfahrens ist die Möglichkeit sowohl Ester und Carboxylate als auch Amide als Ausgangsverbindungen einsetzen zu können.

Das erfindungsgemäße Verfahren kann unter Verwendung der nachfolgend beschriebenen Reaktionsbedingungen und Reaktanten durchgeführt werden. Hierbei können in der Ausgangsverbindung der Formel II und dem Produkt der Formel II alle bevorzugten Reste R¹-R⁴ mit allen anderen bevorzugten Resten R¹-R⁴ kombiniert werden.

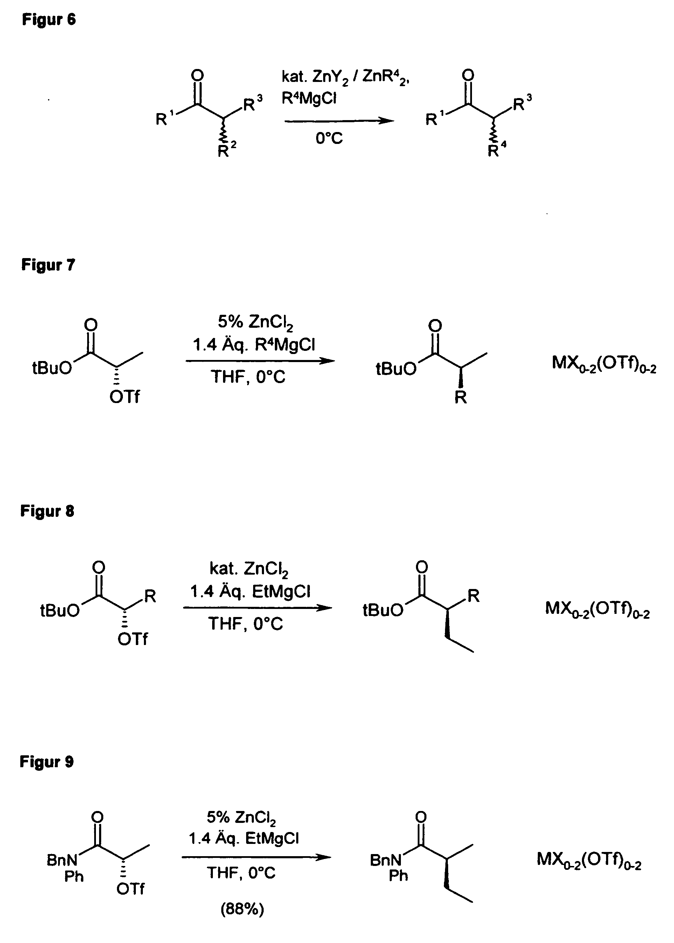

Das Reaktionsschema des erfindungsgemäßen Verfahrens ist in Figur 6 dargestellt. Als Ausgangsverbindungen können hierbei Verbindungen der allgemeinen Formel II eingesetzt werden. Die Verbindungen der Formel II weisen ein chirales Zentrum in α-Stellung zur Carbonylgruppe auf. Der Reaktionsmechanismus des erfindungsgemäßen Verfahrens stellt formal eine SN2-Reaktion dar, wobei die Abgangsgruppe, die sich in α-Stellung zur Carbonylgruppe befindet, unter Inversion der Stereokonfiguration nukleophil substituiert wird. Folglich verläuft das erfindungsgemäße Verfahren unter Erhalt der Stereoinformation.

Der Ausdruck "Erhalt der Stereoinformation" bedeutet hierbei, dass ein Enantiomer der Verbindung der Formel II unter Inversion der Stereokonfiguration in genau ein Enantiomer der α-Alkylcarbonylverbindung der Formel I übergeführt wird. Folglich entspricht die Enantiomerenreinheit bzw. der Enantiomerenüberschuss der hergestellten α-Alkylcarbonylverbindung der Enantiomerenreinheit der eingesetzten Ausgangsverbindung.

Der Ausdruck "Inversion der Stereokonfiguration" in α-Stellung zur Carbonylgruppe bedeutet, dass bei Substitution von R² durch R⁴ eine Inversion der Konfiguration am Kohlenstoffatom in α-Stellung zur Carbonylgruppe stattfindet, so dass R⁴ in Bezug auf die Papierebene "nach vorne steht", wenn R² in der Ausgangsverbindung der Formel II "nach hinten steht" oder R⁴ "nach hinten steht", wenn R² in der Ausgangsverbindung der Formel II "nach vorne steht".

Bevorzugterweise werden die Verbindungen der Formel II mit einem Enantiomerenüberschuss von >95 % *ee*, weiter bevorzugt >96 % *ee*, noch weiter bevorzugt >97 % *ee*, noch weiter bevorzugt >98 % *ee* und am meisten bevorzugt >99 % *ee* eingesetzt.

Für das erfindungsgemäße Verfahren sind alle Ausgangsverbindungen gemäß Formel II geeignet. Verfahren zur Herstellung dieser chiralen Verbindungen sind dem Fachmann bekannt. Zusätzlich bevorzugt werden die Verbindungen der Formel II aus natürlich vorkommenden chiralen Verbindungen hergestellt. Insbesondere geeignet sind natürlich vorkommende chirale Säuren, wie beispielsweise Aminosäuren oder die L-Milchsäure. Bei Verwendung von Aminosäuren wird zunächst gemäß dem in Figur 4 dargestellten und im vorangegangenen beschriebenen Verfahren die α-Hydroxysäure hergestellt. Diese kann dann, wie beispielsweise auch die L-Milchsäure oder die anderen Säuren aus Figur 3, verestert werden. Anschließend wird die Hydroxylgruppe in α-Stellung in eine Abgangsgruppe umgewandelt, um eine Verbindung der allgemeinen Formel II zu erhalten.

Verfahren zur Herstellung von Verbindungen der allgemeinen Formel II sind dem Fachmann bekannt. So sind eine Vielzahl an α-Hydroxysäuren, -ester und amide kommerziell erhältlich, beispielsweise der D-Milchsäure-*tert*-Butylester (*Fluka*), oder können mittels der entsprechenden Fachliteratur, beispielsweise über Veresterungsverfahren (Esterification, Otera Junzo, Wiley-VCH, Weinheim, 2003, p. 1) dargestellt werden. Verfahren zur Umwandlung der Hydroxyfunktion in eine Abgangsgruppe sind ebenfalls bekannt, beispielsweise in einen Trifluormethansulfoxyester (Dragovich et al. J. Med. Chem., 2003, 46, 4572), wobei diese teilweise auch kommerziell erhältlich sind, wie beispielsweise das Triflat des L-Milchsäureethylesters (*Fluka, Aldrich*)

Besonders bevorzugt als Ausgangsverbindungen für die Herstellung von Verbindungen der Formel II sind Carbonylverbindungen die als "chiral pool" bekannt sind und natürlich vorkommende Verbindungen darstellen. Besonders bevorzugte Verbindungen zur Herstellung von Verbindungen der Formel II sind hierbei: Aminosäuren, L-Milchsäure, L-Mandelsäure, L-Äpfelsäure, L-Weinsäure. Diese Verbindungen werden durch Veresterung und Einführen einer Abgangsgruppe in die α-Stellung der Carbonylverbindung in die Ausgangsverbindungen der allgemeinen Formel II übergeführt (α*-Hydroxy Acids in* Enantioselective Syntheses, 1997, Coppola G.M., Schuster H. F., VCH, Weinheim, p. 1).

Die Verbindungen der allgemeinen Formel II weisen die Reste R¹ (mit den Resten R⁵ und R⁶), R², sowie R³ auf.

R¹ kann hierbei die Carbonylverbindung zu einem Carboxylat, einem Ester oder einem Amid ergänzen. Bevorzugterweise ist R¹ gleich OM, O-R⁵ oder NR⁵R⁶, wobei M ein Metallion, bevorzugterweise Natrium oder Kalium, und R⁵ und R⁶ unabhängig voneinander einen geradkettigen oder verzweigten Alkylrest, Alkenylrest, Arylalkylrest, Arylrest oder Heteroarylrest darstellen können. Bevorzugterweise weist hierbei ein Alkylrest 1-15 Kohlenstoffatome, ein Alkenylrest 2-15 Kohlenstoffatome, ein Arylalkylrest 5-15 Kohlenstoffatome, ein Arylrest 5-10 Kohlenstoffatome und ein Heteroarylrest 5-8 Kohlenstoffatome, sowie 1-2 Heteroatome, ausgewählt aus O, N, P und S, auf. Weiter bevorzugt weist ein Alkylrest 1-10, weiter bevorzugt 1-7 Kohlenstoffatome, ein Alkenylrest 2-10, weiter bevorzugt 2-7 Kohlenstoffatome, ein Arylalkylrest 5-10, weiter bevorzugt 5-8 Kohlenstoffatome, ein Arylrest 5-8, weiter bevorzugt 5-6 Kohlenstoffatome und ein Heteroarylrest 5-7, weiter bevorzugt 5-6 Kohlenstoffatome, sowie 1-2 Heteroatome, ausgewählt aus O, N, P und S, weiter bevorzugt ausgewählt aus O, N und S, auf. Weiter bevorzugt ist R¹ gleich O-R⁵. Noch weiter bevorzugt ist hierbei R⁵ gleich Methyl, Ethyl, *i*Propyl, *i*Butyl, Benzyl oder *tert*Butyl, weiter bevorzugt *tert*Butyl.

R² stellt eine Abgangsgruppe dar, bevorzugterweise ausgewählt aus der Gruppe bestehend aus: Sulfonatgruppen, Phosphatgruppen, Carboxylatgruppen, Carbamatgruppen, Carbonatgruppen und Halogeniden. Der Ausdruck "Sulfonatgruppen" betrifft alle von der Sulfonsäure abgeleiteten Gruppen. Bevorzugt sind hierbei Alkylsulfonsäuregruppen, weiter bevorzugt halogenierte Alkylsulfonsäuregruppen mit 1-9 Halogensubstituenten, weiter bevorzugt mit 1-9 Fluoratomen. Noch weiter bevorzugt sind perfluorierte Alkylsulfonsäuregruppen, am meisten bevorzugt sind Trifluormethansulfonsäuregruppe (Triflat) und Perfluorbutansulfonsäuregruppe (Nonaflat). Der Ausdruck "Phosphatgruppen" betrifft alle von der Phosphorsäure abgeleiteten Gruppen. Bevorzugt sind hierbei Alkyl- und Arylphosphatgruppen, weiter bevorzugt halogenierte Alkyl- und Arylphosphatgruppen mit 1-14 Halogensubstituenten, weiter bevorzugt mit 1-14 Fluoratomen. Noch weiter bevorzugt sind perfluorierte Alkyl- und Arylphosphatgruppen. Der Ausdruck "Carboxylatgruppe" betrifft alle von der Carbonsäure abgeleiteten Gruppen. Bevorzugt sind hierbei Alkyl- und Arylcarboxylatgruppen, weiter bevorzugt halogenierte Alkyl- und Arylcarboxylatgruppen mit 1-10 Halogensubstituenten, weiter bevorzugt mit 1-10 Fluoratomen. Noch weiter bevorzugt sind perfluorierte Alkyl- und Arylcarboxylatgruppen. Der Ausdruck "Carbamatgruppe" betrifft alle von der Carbaminsäure abgeleiteten Gruppen. Bevorzugt sind hierbei Alkyl- und Arylcarbamatgruppen, weiter bevorzugt halogenierte Alkyl- und Arylcarbamatgruppen mit 1-14 Halogensubstituenten, weiter bevorzugt mit 1-14 Fluoratomen. Noch weiter bevorzugt sind perfluorierte Alkyl- und Arylcarbamatgruppen. Der Ausdruck "Carbonatgruppe" betrifft alle von der Kohlensäure abgeleiteten Gruppen. Bevorzugt sind hierbei Alkyl- und Arylcarbonatgruppen, weiter bevorzugt halogenierte Alkyl- und Arylcarbonatgruppen mit 1-10 Halogensubstituenten, weiter bevorzugt mit 1-10 Fluoratomen. Noch weiter bevorzugt sind perfluorierte Alkyl- und Arylcarbonatgruppen. Die bevorzugte Halogenid-Abgangsgruppe ist Chlorid. Bevorzugterweise stellt R³ einen geradkettigen oder verzweigten Alkylrest, Alkenylrest, Arylalkylrest, oder Arylrest dar, der 1-7, weiter bevorzugt 1-5, noch weiter bevorzugt 1-3 Heteroatome, ausgewählt aus O, N und S, als Substituenten aufweisen oder von den Heteroatomen unterbrochen werden kann. Der Ausdruck, dass ein Alkylrest, Alkenylrest Arylalkylrest, oder Arylrest durch Heteroatome "unterbrochen werden kann" bedeutet, dass sich die Heteroatome zwischen den Kohlenstoffatomen in der Alkylkette sowie im Arylring befinden können. Falls Heteroatome als Substituenten enthalten sind, können diese bevorzugt auch Schutzgruppen tragen, beispielsweise falls es sich um Hydroxyl- oder Aminogruppen handelt, um Nebenreaktionen mit der Organometallverbindung zu vermeiden.

Bevorzugterweise weist in Bezug auf R³ ein Alkylrest 1-15 Kohlenstoffatome, ein Alkenylrest 2-15 Kohlenstoffatome, ein Arylalkylrest 5-15 Kohlenstoffatome, ein Arylrest 5-10 Kohlenstoffatome und ein Heteroarylrest 5-8 Kohlenstoffatome, sowie 1-2 Heteroatome, ausgewählt aus O, N, P und S, auf. Weiter bevorzugt weist ein Alkylrest 1-10, weiter bevorzugt 1-7 Kohleristoffatome, ein Alkenylrest 2-10, weiter bevorzugt 2-7 Kohlenstoffatome, ein Arylalkylrest 5-10, weiter bevorzugt 5-8 Kohlenstoffatome, ein Arylrest 5-8, weiter bevorzugt 5-6 Kohlenstoffatome und ein Heteroarylrest 5-7, weiter bevorzugt 5-6 Kohlenstoffatome, sowie 1-2 Heteroatome, ausgewählt aus O, N, P und S, weiter bevorzugt ausgewählt aus O, N und S, auf. Weiter bevorzugt ist R¹ gleich O-R⁵. Noch weiter bevorzugt ist hierbei R⁵ gleich Methyl, Ethyl, *i*Propyl, *i*Butyl, Benzyl oder *tert*Butyl, weiter bevorzugt *tert*Butyl.

Um die Abgangsgruppe in α-Stellung der Carbonylgruppe zu substituieren kann eine "nukleophile Alkylgruppe" unter Verwendung eines Magnesium-Grignard-Reagenzes und ZnY₂ hergestellt werden. Hierbei wird postuliert, dass die aktive Spezies auf einem Trialkylzink(II)at-Komplex bzw. einem Trialkenylzink(II)at-Komplex, bzw. einem Triarylzink(II)at-Komplex, R⁴₃ZnMgX basiert. R⁴₃ZnMgX entsteht dabei aus R⁴MgX und R⁴₂Zn, welches zunächst aus ZnY₂ und R⁴MgX gebildet wird (Ishihara et al., J. Am. Chem. Soc., 2006, 128, 9998). Für das erfindungsgemäße Verfahren sind jegliche Magnesium-Grignard-Reagenzien geeignet. Besonders bevorzugt sind Magnesium-Grignard-Reagenzien der allgemeinen Formel R⁴MgX, worin X bevorzugt ein Halogen; weiter bevorzugt Chlor, Brom oder Jod, noch weiter bevorzugt Chlor darstellt. Für das erfindungsgemäße Verfahren sind jegliche Zinksalze geeignet. Besonders bevorzugt sind Zinksalze der allgemeinen Formel ZnY₂, worin Y bevorzugt ein Sulfonat, ein Sulfat, ein Carboxylat, ein Alkoholat, ein Nitrat, ein Chalkogen oder ein Halogen, weiter bevorzugt Chlor, Brom, Iod, noch weiter bevorzugt Chlor darstellt. Des Weiteren ist es auch möglich, an Stelle von ZnY₂ direkt das Zinkorganyl ZnR⁴₂ zu verwenden, da aus ZnR⁴₂ und R⁴MgX, wie oben beschrieben, die aktive Spezies R⁴₃ZnMgX entsteht.

R⁴ stellt bevorzugt einen geradkettigen oder verzweigten Alkylrest, Alkenylrest, Arylalkylrest, Arylrest oder Heteroarylrest dar. Bevorzugterweise weist ein Alkylrest 1-15 Kohlenstoffatome, ein Alkenylrest 2-15 Kohlenstoffatome, ein Arylalkylrest 5-15 Kohlenstoffatome, ein Arylrest 5-10 Kohlenstoffatome und ein Heteroarylrest 5-8 Kohlenstoffatome, sowie 1-2 Heteroatome, ausgewählt aus O, N, P und S, auf. Weiter bevorzugt weist ein Alkylrest 1-10, weiter bevorzugt 1-7 Kohlenstoffatome, ein Alkenylrest 2-10, weiter bevorzugt 2-7 Kohlenstoffatome, ein Arylalkylrest 5-10, weiter bevorzugt 5-8 Kohlenstoffatome, ein Arylrest 5-8, weiter bevorzugt 5-6 Kohlenstoffatome und ein Heteroarylrest 5-7 Kohlenstoffatome, sowie 1-2 Heteroatome, ausgewählt aus O, N, P und S, auf. Besonders bevorzugt stellt R⁴ eine Methyl-, Ethyl-, *i*Propyl-, *n*Butyl-, *i*Butyl-, *s*Butyl-, Cyclohexyl-, Octyl-, Lauryl-, Allyl-, Metallyl-, Vinyl-, Phenyl-, Benzyl-,3-Benzyloxypropyl- und 3-Benzyloxy-2-methylpropylgruppe dar.

Das Magnesium-Grignard-Reagenz wird bevorzugterweise in Mengen zwischen 1 und 2, weiter bevorzugt 1,2-1,6 Äquivalenten (Äq.), bezogen auf die Stoffmenge der Ausgangsverbindung der Formel 11, eingesetzt.

Um das gewünschte Organometall-Reagenz herzustellen, wird ZnY₂ in trockener Form verwendet. Hierbei werden bevorzugt zwischen 0,5 - 25 Gew.-% ZnY₂, bezogen auf die Menge an Ausgangsstoff der Formel II, verwendet. Das ZnY₂ kann hierbei auch von Lösungsmittelmolekülen, beispielsweise TMEDA (Tetramethylethylendiamin), komplexiert sein. Als geeignete Zink-Katalysatoren haben sich auch Zn(OA_{c})₂ und ZnCl₂ TMEDA bei 0°C sowie Zn(OTf)₂ bei -20°C erwiesen.

Zusätzlich bevorzugt werden für das erfindungsgemäße Verfahren Lösungsmittel verwendet, die für Grignard-Reaktionen geeignet sind. Diese Lösungsmittel sind dem Fachmann bekannt. Bevorzugterweise werden Tetrahydrofuran, 2-Methyltetrahydrofuran, Diethylether oder Gemische aus diesen beiden Lösungsmitteln verwendet. Aufgrund der Hydrolyse von Organometallverbindungen durch Wasser sollte ein möglichst wasserfreies organisches Lösungsmittel verwendet werden.

Das erfindungsgemäße Verfahren wird bevorzugterweise bei Temperaturen zwischen -30°C und 30°C, weiter bevorzugt zwischen -15°C und 10°C, weiter bevorzugt zwischen -10°C und 10°C, noch weiter bevorzugt zwischen -5°C und 5°C und am meisten bevorzugt bei 0°C, durchgeführt. Die Reaktionsdauer beträgt bevorzugterweise zwischen 10 Minuten und 3 Stunden, noch weiter bevorzugt zwischen 1 Stunde und 2 Stunden. Das exakte Reaktionsende kann gegebenenfalls durch übliche Verfahren bestimmt werden. Geeignete Verfahren sind hierbei Messmethoden wie GC-MS (Gaschromatographie gekoppelt mit Massenspektrometrie) oder NMR (Nuclear Magnetic Resonance)-Spektroskopie.

Die Durchführung des erfindungsgemäßen Verfahrens wird in den Beispielen weiter beschrieben und umfasst einen ersten Schritt, in dem das trockene Zink(II)salz ZnY₂ in trockenem Lösungsmittel gelöst und anschließend gekühlt wird (beispielsweise auf 0°C). Das Lösungsmittel kann auch vor der Zugabe des Zink(II)salzes ZnY₂ gekühlt werden. Anschließend kann der Ausgangsstoff der Formel II und nachfolgend das Grignard-Reagenz oder in umgekehrter Reihenfolge zugegeben werden. Die Aufreinigung des Reaktionsgemisches kann nach üblichen Verfahren beispielsweise durch Destillation erfolgen. Die Charakterisierung der Produkte kann nach üblichen Verfahren erfolgen, beispielsweise unter Verwendung von GC-MS oder NMR-Spektroskopie.

### Beschreibung der Figuren

### Verwendete Abkürzungen:

*n*-BuLi = *n*-Buthylithium; THF = Tetrahydrofuran; NaHMDS = Natrium-Hexamethyldisilazid; Mel = Methyliodid; KOH = Kaliumhydroxid; NaH = Natriumhydrid; HMPA = Hexamethylphosphoramid und LiOH = Lithiumhydroxid; Tf = Triflat;
**Figur 1** zeigt das Oxazolidinon-Verfahren zur Herstellung von α-Alkylcarbonylverbindungen. Hierbei erfolgt unter Verwendung von *n*-BuLi und n-C₅H₁₂COCl eine Acylierungsreaktion der Ausgangsverbindung 5. An die dadurch entstehende Verbindung 7 kann in der Enolatform eine Methylgruppe addiert und anschließend in wässriger alkalischer Lösung die α-Alkylcarbonylverbindung erhalten werden.
**Figur 2** zeigt das Oppolzer-Sultam-Verfahren zur Herstellung von α-Alkylcarbonylverbindungen. Hierbei wird das Opolzer-Sultam (Verbindung 12) als chirales Hilfsreagenz eingesetzt und nach Addition einer Carbonylverbindung an das chirale Hilfsreagenz kann eine stereoselektive Addition einer Methylgruppe in α-Stellung der Carbonylverbindung erfolgen und anschließend, unter Verwendung von Lithiumhydroxid, die freie α-Alkylcarbonylverbindung erhalten werden.
**Figur 3** zeigt natürlich vorkommende enantiomerenreine α-Hydroxysäuren (chiral pool), die zur Herstellung der Ausgangsverbindungen gemäß Formel II verwendet werden können. Verbindung 1 = L-Milchsäure; Verbindung 2 = L-Mandelsäure; Verbindung 3 = L-Äpfelsäure; Verbindung 4 = L-Weinsäure.
**Figur 4** zeigt die Herstellung von α-Hydroxysäuren aus chiralen Aminosäuren unter Verwendung von Schwefelsäure und Natriumnitrit.
**Figur 5** zeigt die stereoselektive Umsetzung von α-Trifluormethansulfoxyestern nach Larchevêque et al. mit Dialkylcupraten bei tiefen Temperaturen und unter Verwendung einer stöchiometrischen Menge an Kupfersalz.
**Figur 6** zeigt das erfindungsgemäße Verfahren unter Verwendung von ZnY₂ und R⁴MgCl bei 0°C.
**Figur 7** zeigt das erfindungsgemäße Verfahren unter Verwendung einer Trifluorsulfonsäuregruppe als Abgangsgruppe und die Verwendung von 5 % ZnCl₂ sowie 1,4 Äquivalenten (Äq.) R⁴MgCl in THF bei 0°C.
**Figur 8** zeigt die Verwendung von *tert*-Butylestern mit Triflat-Abgangsgruppen in α-Stellung zur Carbonylgruppe. Diese Verbindungen werden unter Verwendung von EtMgCl und ZnCl₂ bei 0°C zum α-Alkylester umgesetzt.
**Figur 9** zeigt die Verwendung eines Benzylphenylamids mit einer Triflat-Abgangsgruppe in α-Stellung zur Carbonylverbindung. Diese Ausgangsverbindung wird mit EtMgCl und ZnCl₂ zum entsprechenden α-Alkylamid umgesetzt.

### Beispiel 1: Herstellung von optisch aktivem (S)-2-Trifluormethansulfonyloxy-propion-säure-tert-butylester

In einem 500 ml Rundkolben wurde unter Argon 3,00 g L-Mitchsäure-*tert*-butylester (*ee* >99 %) in 165 ml absolutem Dichlormethan gelöst und auf 0°C gekühlt. Anschließend wurde nacheinander erst 3,10 ml 2,6-Lutidin und dann langsam 4.15 ml Trifluormethansulfonsäureanhydrid zugegeben und die Reaktion 50 min bei 0°C gerührt. Daraufhin wurde die Reaktionsmischung mit 400 ml Petrolether verdünnt, 4 mal mit 250 ml einer 3 : 1 Mischung aus gesättigter wässriger Natriumchlorid-Lösung und 1 N Salzsäure gewaschen, über MgSO₄ getrocknet und am Vakuum eingeengt. Der Rückstand wurde auf einen Kieselgelfilter aufgetragen und von diesem mit einer 1 : 1 Mischung aus Petrolether und Dichlormethan heruntergespült. Nach Entfernen des Lösungsmittels erhielt man den reinen (S)-2-Trifluor-methansulfonyloxypropionsäure-*tert-*butylester (*ee* >99 %) in einer Ausbeute von 90%.

### Beispiel 2: Herstellung von optisch aktivem (S)-2-Methylhexansäure-tert-butylester

In ein 10 ml Schlenkrohr wurde unter Argon 3,5 mg trockenes Zinkchlorid in 3,0 ml absolutem Tetrahydrofuran gelöst und auf 0°C gekühlt. Anschließend wurde nacheinander 279 mg (*S*)-2-Trifluormethansulfonyloxypropionsäure-*tert*-butylester (*ee* >99 %) und 0,700 ml nButyl-magnesiumchlorid (2,00 M-Lösung in THF) zugegeben und die Reaktion 3 h bei 0°C gerührt. Daraufhin wurde die Reaktionsmischung mit Petrolether verdünnt, erst Wasser und dann gesättigte Ammoniumchlorid-Lösung zugegeben und die organische Phase abgetrennt. Diese wurde direkt auf einen Kieselgelfilter aufgetragen und mit Petrolether gewaschen. Anschließend wurde das Produkt mit einer Petrolether/Diethylether-Mischung (10:1) von dem Filter heruntergespült und vom Lösungsmittel befreit. Man erhielt den reinen (*S*)-2-Methylhexansäure-*tert*-butylester (Eintrag 3 in Tabelle 1) (*ee* >99 %) in quantitativer Ausbeute.

### Beispiel 3: Herstellung von optisch aktivem (2S)-2,3-Dimethyibutansäure-tert-butylester

Die Herstellung des Trifluormethansulfonyloxypropionsäure-*tert*-butylester erfolgte wie in Beispiel 1. In ein 10 ml Schlenkrohr wurde unter Argon 7,0 mg trockenes Zinkchlorid in 3,0 ml absolutem Tetrahydrofuran gelöst und auf 0°C gekühlt. Anschließend wurde nacheinander 279 mg (*S*)-2-Trifluormethansulfonyloxypropionsäure-*tert*-butylester (*ee* >99 %) und 0,820 ml *iso*-Propylmagnesiumchlorid (1,70 M-Lösung in THF) zugegeben und die Reaktion 3 h bei 0°C gerührt. Daraufhin wurde die Reaktionsmischung mit Petrolether verdünnt, erst Wasser und dann gesättigte Ammoniumchlorid-Lösung zugegeben und die organische Phase abgetrennt. Diese wurde direkt auf einen Kieselgelfilter aufgetragen und mit Petrolether gewaschen. Anschließend wurden die Produkte mit einer Petrolether/Diethylether-Mischung (10:1) von dem Filter heruntergespült und vom Lösungsmittel befreit. Man erhielt 98 mol-% (2*S*)-2,3-Dimethylbutansäure-*tert*-butylester (Eintrag 2 in Tabelle 1) (*ee* >99%) und 2 mol-% Propionsäure-*tert*-butylester. Die Zielverbindung ließ sich mittels einer KugelrohrDestillation aufreinigen.

Sowohl in Beispiel 2, als auch in Beispiel 3 lassen sich die Produkte bei einer Synthese in größerem Maßstab problemlos mittels einer fraktionierten Destillation direkt isolieren.

### Beispiel 4: Zusammenfassung aller Beispiele mit tert-Butylestern als Ausgangsverbindungen

Die Herstellung der in der nachfolgenden Tabelle 1 aufgeführten α-Alkylcarbonylverbindungen erfolgte analog den Beispielen 1 und 2 (siehe auch Figur 7).

**Tabelle 1**

| Eintrag | R | Ausbeute*^{a}* | *ee* (%) |
|---|---|---|---|
| 1 | Et | quantitativ | >99 |
| 2 | *i*Pr | 98 | >99 |
| 3 | *n*Bu | quantitativ | >99 |
| 4 | *i*Bu | quantitativ^{b} | >99 |
| 5 | *s*Bu | 96 | >99 |
| 6 | Cy | 90*^{c}* | >99 |
| 7 | Oct | quantitativ | >99 |
| 8 | Lauryl | quantitativ | >99 |
| 9 | Bn | quantitativ | >99 |
| 10 | 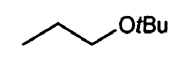 | 94 | >99 |
| 11 | 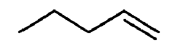 | quantitativ | >99 |

| | | | |
|---|---|---|---|
| *^{a}*Isolierte Ausbeute, *^{b}*20 % ZnCl₂, *^{c}*10 % ZnCl₂ Abkürzungen in der Tabelle: Et = Ethyl, *i*Pr = *iso*- Propyl, *n*Bu = *n*-Butyl, *i*Bu =*iso*-Butyl, *s*Bu = sec-Butyl, Cy = Cyclohexyl, Oct = Octyl, Bn = Benzyl; | | | |

Tabelle 1 zeigt, dass sowohl unverzweigte, als auch verzweigte, sowie aromatische Grignard-Reagenzien zur Synthese von optisch reinen α-Alkylcarbonylverbindungen geeignet sind. Insbesondere vorteilhaft ist hierbei die Möglichkeit, enantiomerenreine α-Alkylcarbonylverbindungen mit sekundären Alkylresten in der α-Stellung darstellen zu können.

### Beispiel 5: Zusammenfassung der Beispiele unter Verwendung von unterschiedlichen tert-Butylestern

Mit dem erfindungsgemäßen Verfahren können unterschiedlichste Carbonylverbindungen mit Abgangsgruppen als Ausgangsverbindungen zur Synthese von α-Alkylcarbonylverbindungen eingesetzt werden. Dies wird durch die in Tabelle 2 dargestellten Ausgangsverbindungen der allgemeinen Formel II gezeigt. Das zugehörige Reaktionsschema ist in Figur 8 dargestellt. Die Synthesen wurden gemäß der Vorschrift aus Beispiel 2 durchgeführt.

**Tabelle 2**

| Eintrag | Substrat | ZnCl₂ (mol-%) | Ausbeute (%) |
|---|---|---|---|
| 1 | 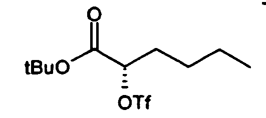 | 5 | quantitativ |
| 2 | 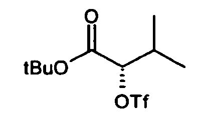 | 15 | quantitativ |
| 3 | 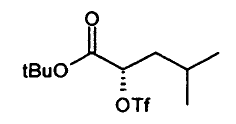 | 10 | quantitativ |
| 4 | 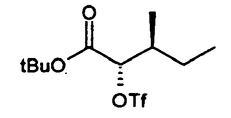 | 20 | quantitativ |
| 5 | 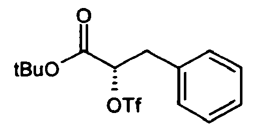 | 5 | quantitativ |
| 6 | 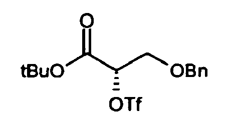 | 20 | quantitativ |
| 7 | 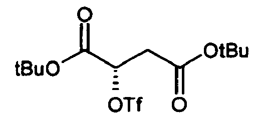 | 5 | quantitativ |

Die in Tabelle 2 dargestellten Ausgangsverbindungen lassen sich alle einfach aus natürlich vorkommenden enantiomerenreinen Verbindungen (Aminosäuren, Einträge 1-6; Äpfelsäure, Eintrag 7) synthetisieren. Selbst β-verzweigte α-Alkylcarbonylverbindungen sind als Ausgangsverbindungen geeignet, wodurch das erfindungsgemäße Verfahren einen Zugang zu einer unbegrenzten Zahl an optisch aktiven α-Alkylcarbonylverbindungen bietet.

### Beispiel 6: Verwendung von α-Trifluorsulfoxyamiden als Ausgangsverbindungen

Figur 9 zeigt die Verwendung von α-Trifluormethansulfoxyamiden als Ausgangsverbindungen, die aus den entsprechenden α-Hydroxyamiden nach dem in Beispiel 1 beschriebenen Verfahren hergestellt wurden. Die Synthese des chiralen α-Alkylamids erfolgte unter Verwendung der in Beispiel 2 beschriebenen Vorschrift. Bei der in Figur 9 dargestellten Reaktion konnte eine Ausbeute von 88 % erreicht werden.

### Beispiel 7: Herstellung und Umsetzung von weiteren Amidverbindungen

Die Umsetzung erfolgte wie in Beispielen 1 und 2 (siehe Figur 9).

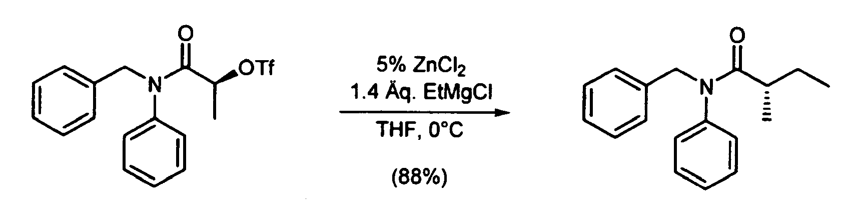

### Beispiel 8: Herstellung und Umsetzung von Esterverbindungen mit einer Nonaflat-Abgangsgruppe:

### Beispiel 8a: Herstellung von optisch aktivem (S)-2-(Nonafluorbutan-1-sulfonyloxy)-propionsäure-tert-butylester

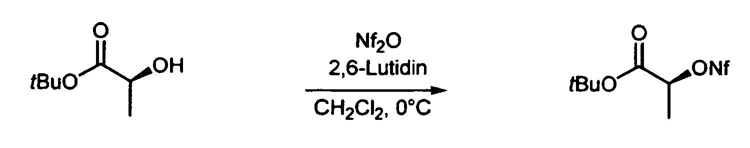

Die Herstellung des Nonafluorbutansulfonyloxypropionsäure-*tert*-butylesters erfolgte mit Nonafluorbutansulfonsäureanhydrid wie in Beispiel 1 mit einer Ausbeute von 82% (*ee* >99%).

### Beispiel 8b: Herstellung von optisch (S)-2-Methylhexansäure-tert-butylester

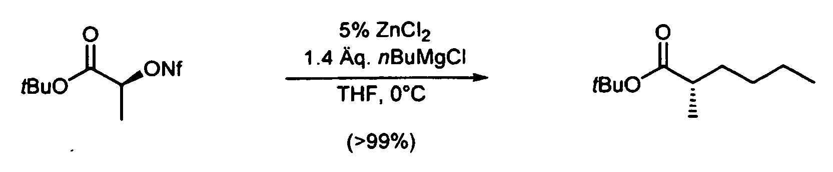

Die Herstellung von (*S*)-2-Methylhexansäure-*tert*-butylester mit (*S*)-2-(Nonafluorbutan1-sulfonyloxy)-propionsäure-*tert*-butylester erfolgte wie in Beispiel 2 mit einer quantitativen Ausbeute (*ee* >99%).

### Beispiel 9: Carboxylatverbindungen wurden folgendemaßen hergestellt und

### umgesetzt:

Herstellung des Triflats der Milchsäure:

### Beispiel 9a: Herstellung von optisch aktiver (S)-2-Trifluormethansulfonyloxypropionsäure

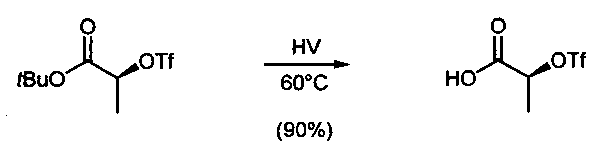

In einem 10 ml Rundkolben wurde 1,00 g (*S*)-2-Trifluormethansulfonyloxypropionsäure-*tert*-butylester (*ee* >99 %) unter Hochvakuum (0.01 mbar) bei 60°C erhitzt. Der leicht gelbliche Rückstand enthielt reine (*S*)-2-Trifluormethansulfonyloxypropionsäure (*ee* 99 %) in einer Ausbeute von 90%.

### Beispiel 9b: Herstellung von optisch aktiver (S)-2-Mothylhexansäure

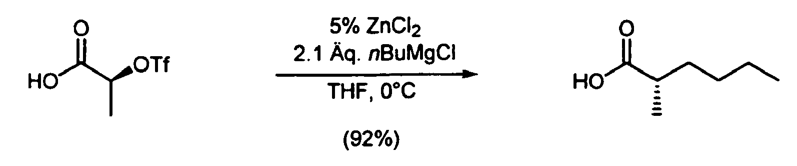

In ein 10 ml Schlenkrohr wurde unter Argon 5,0 mg trockenes Zinkchlorid in 3,0 ml absolutem Tetrahydrofuran gelöst und auf 0°C gekühlt. Anschließend wurde nacheinander 222 mg (*S*)-2-Trifluormethansulfonyloxypropionsäure (*ee* 99 %) und 1,05 ml *n*Butyl-magnesiumchlorid (2,00 M-Lösung in THF) zugegeben und die Reaktion 3 h bei 0°C gerührt. Daraufhin wurde die Reaktionsmischung mit Petrolether verdünnt, gesättigte Natriumcarbonat-Lösung zugegeben und die organische Phase abgetrennt. Die wässrige Phase wurde mit einer 2 M wässrigen HCl-Lösung angesäuert, mit NaCl gesättigt und 3 mal mit 10 ml Diethylether extrahiert. Die vereinigten organischen Phasen wurden über MgSO₄ getrocknet und am Vakuum eingeengt. Man erhielt die reine (*S*)-2-Methylhexansäure (*ee* 99 %) in 92%iger Ausbeute.

Ebenso wie in Beispiel 8 lassen sich auch in Beispiel 10 die Produkte bei einer Synthese in größerem Maßstab problemlos mittels einer fraktionierten Destillation direkt isolieren.

## Patentansprüche

1. Verfahren zur Herstellung von Verbindungen der Formel 1: 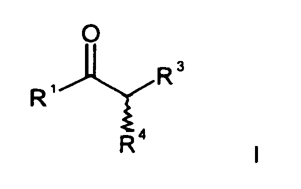 worin eine Verbindung der Formel II, unter Inversion der Stereokonfiguration am α-Carbonylkohlenstoffatom, 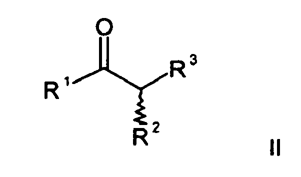 mit R⁴MgX und ZnY₂ oder R⁴MgX und ZnR⁴₂ zu einer Verbindung gemäß Formel I umgesetzt wird,
worin R¹ gleich OM, O-R⁵ oder NR⁵R⁶ sein kann, wobei M ein Metallion und R⁵ und R⁶ unabhängig voneinander einen geradkettigen oder verzweigten Alkylrest, Alkenylrest, Arylalkylrest, Arylrest oder Heteroarylrest darstellen können,
worin R² eine Sulfonatabgangsgruppe, Phosphatabgangsgruppe, Carboxylatabgangsgruppe, Carbamatabgangsgruppe, Carbonatabgangsgruppe oder ein Halogenid ist,
worin R³ einen geradkettigen oder verzweigten Alkylrest, Alkenylrest, Arylalkylrest, Arylrest oder Heteroarylrest darstellt, der 5-7 Kohlenstoffatome, sowie 1-2 Heteroatome, ausgewählt aus O, N, P und S, als Substituenten aufweisen oder von den Heteroatomen unterbrochen sein kann,
worin R⁴ einen geradkettigen oder verzweigten Alkylrest, Alkenylrest, Arylalkylrest, Arylrest oder Heteroarylrest darstellt,
worin bei R¹ bis R⁶ ein Alkylrest 1-15 Kohlenstoffatome, ein Alkenylrest 2-15 Kohlenstoffatome, ein Arylalkylrest 5-15 Kohlenstoffatome, ein Arylrest 5-10 Kohlenstoffatome und ein Heteroarylrest 5-8 Kohlenstoffatome, sowie 1-2 Heteroatome, ausgewählt aus O, N, P und S, aufweisen kann,
worin X ein Halogen darstellt, und
worin Y ein Sulfonat, ein Sulfat, ein Carboxylat, ein Alkoholat, ein Nitrat, ein Chalkogen oder ein Halogen darstellen kann.

2. Verfahren gemäß Anspruch 1, **dadurch gekennzeichnet, dass** das Verfahren bei Temperaturen von mehr als -30°C durchgeführt wird.

3. Verfahren gemäß einem der vorangegangenen Ansprüche, **dadurch gekennzeichnet, dass** die Verbindungen der Formel II mit einem Enantiomerenüberschuss (*ee*) von mehr als 95 % eingesetzt und die Verbindungen der Formel I mit einem Enantiomerenüberschuss von mehr als 95 % hergestellt werden.

4. Verfahren gemäß einem der vorangegangenen Ansprüche, **dadurch gekennzeichnet, dass** 0,5-25 Gew.-% ZnY₂ oder ZnR⁴₂, bezogen auf die Menge der Verbindung der Formel II, verwendet werden.

5. Verfahren gemäß einem der vorangegangenen Ansprüche, **dadurch gekennzeichnet, dass** als Lösungsmittel für die Reaktion Tetrahydrofuran, 2-Methyltetrahydrofuran oder Diethylether bzw. ein Gemisch aus diesen Lösungsmitteln verwendet wird.

6. Verfahren gemäß einem der vorangegangenen Ansprüche, **dadurch gekennzeichnet, dass** ein Alkylrest 1-10 Kohlenstoffatome, ein Alkenylrest 2-10 Kohlenstoffatome, ein Arylalkylrest 5-10 Kohlenstoffatome, ein Arylrest 5-8 Kohlenstoffatome und ein Heteroarylrest 5-7 Kohlenstoffatome, sowie 1-2 Heteroatome, ausgewählt aus O, N, P und S, umfasst.

7. Verfahren gemäß einem der vorangegangenen Ansprüche, **dadurch gekennzeichnet, dass** R² eine Alkylsulfonsäuregruppe, Phosphatgruppe, Carboxylatgruppe, Carbamatgruppe, Carbonatgruppe oder Halogen darstellt.

8. Verfahren nach Anspruch 7, **dadurch gekennzeichnet, dass** R² eine Triflat- oder Nonaflat-Abgangsgruppe ist.

9. Verfahren nach einem der Ansprüche 1-5, **dadurch gekennzeichnet, dass** R⁴ eine Methyl-, Ethyl-, *i*Propyl-, *n*Butyl-, *i*Butyl-, *s*Butyl-, Cyclohexyl-, Octyl-, Lauryl-, Allyl-, Metallyl-, Vinyl-, Phenyl-, Benzyl-, 3-Benzyloxypropyl- und 3-Benzyloxy-2-methylpropylgruppe darstellt.

10. Verfahren gemäß einem der vorangegangenen Ansprüche, **dadurch gekennzeichnet, dass** 1-2 Äquivalente R⁴MgX , bezogen auf die Stoffmenge der Verbindung der Formel 11, verwendet werden.

11. Verfahren gemäß einem der vorangegangenen Ansprüche, **dadurch gekennzeichnet, dass** die Verbindungen der Formel II aus natürlich vorkommenden, chiralen Verbindungen hergestellt werden.

12. Verfahren gemäß einem der vorangegangenen Ansprüche, **dadurch gekennzeichnet, dass** die Verbindungen der Formel II hergestellt werden aus Verbindungen, ausgewählt aus der Gruppe bestehend aus: Aminosäuren, L-Milchsäure, L-Äpfelsäure, L-Weinsäure, L-Mandelsäure.

## Claims

1. Method for the production of compounds of the formula I: 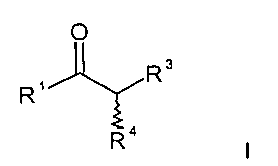 wherein a compound of the formula II, 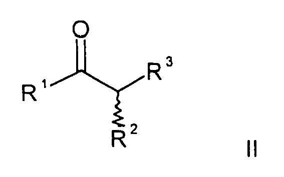 is reacted with R⁴MgX and ZnY₂ or R⁴MgX and ZnR⁴₂, with inversion of the stereoconfiguration at the α-carbonyl carbon atom, to give a compound according to formula I,
in which R¹ may be OM, O-R⁵ or NR⁵R⁶, where M may be a metal ion and R⁵ and R⁶, independently of one another, may be a straight-chain or branched alkyl radical, alkenyl radical, arylalkyl radical, aryl radical or heteroaryl radical,
in which R² is a sulphonate leaving group, phosphate leaving group, carboxylate leaving group, carbamate leaving group, carbonate leaving group or a halide,
in which R³ represents a straight-chain or branched alkyl radical, alkenyl radical, arylalkyl radical, aryl radical or heteroaryl radical which may have 5-7 carbon atoms and 1-2 hetero atoms, selected from O, N, P and S, as substituents or may be interrupted by the hetero atoms,
in which R⁴ represents a straight-chain or branched alkyl radical, alkenyl radical, arylalkyl radical, aryl radical or heteroaryl radical,
in which, in R¹ to R⁶, an alkyl radical may have 1-5 carbon atoms, an alkenyl radical 2-15 carbon atoms, an arylalkyl radical 5-15 carbon atoms, an aryl radical 5-10 carbon atoms and a heteroaryl radical 5-8 carbon atoms and 1-2 hetero atoms, selected from O, N, P and S,
in which X represents a halogen and
in which Y may be a sulphonate, a sulphate, a carboxylate, an alcoholate, a nitrate, a chalcogen or a halogen.

2. Method according to Claim 1, **characterized in that** the method is carried out at temperatures of more than -30°C.

3. Method according to either of the preceding claims, **characterized in that** the compounds of the formula II are used with an enantiomeric excess (*ee*) of more than 95% and the compounds of the formula I are prepared with an enantiomeric excess of more than 95%.

4. Method according to any of the preceding claims, **characterized in that** 0.5 - 25% by weight of ZnY₂ or ZnR⁴₂, based on the amount of the compound of the formula II, are used.

5. Method according to any of the preceding claims, **characterized in that** tetrahydrofuran, 2-methyltetrahydrofuran or diethyl ether or a mixture of these solvents is used as a solvent for the reaction.

6. Method according to any of the preceding claims, **characterized in that** an alkyl radical comprises 1-10 carbon atoms, an alkenyl radical comprises 2-10 carbon atoms, an arylalkyl radical comprises 5-10 carbon atoms, an aryl radical comprises 5-8 carbon atoms and a heteroaryl radical comprises 5-7 carbon atoms and 1-2 hetero atoms, selected from O, N, P and S.

7. Method according to any of the preceding claims, **characterized in that** R² represents an alkylsulphonic acid group, phosphate group, carboxylate group, carbamate group, carbonate group or halogen.

8. Method according to Claim 7, **characterized in that** R² is a triflate or nonaflate leaving group.

9. Method according to any of Claims 1-5, **characterized in that** R⁴ represents a methyl, ethyl, isopropyl, n-butyl, isobutyl, sec-butyl, cyclohexyl, octyl, lauryl, allyl, methallyl, vinyl, phenyl, benzyl, 3-benzyloxypropyl and 3-benzyloxy-2-methylpropyl group.

10. Method according to any of the preceding claims, **characterized in that** 1-2 equivalents of R⁴MgX, based on the amount of the compound in the formula II, are used.

11. Method according to any of the preceding claims, **characterized in that** the compounds of the formula II are produced from naturally occurring, chiral compounds.

12. Method according to any of the preceding claims, **characterized in that** the compounds of the formula II are produced from compounds selected from the group consisting of: amino acids, L-lactic acid, L-malic acid, L-tartaric acid, L-mandelic acid.

## Revendications

1. Procédé de fabrication de composés de formule I : 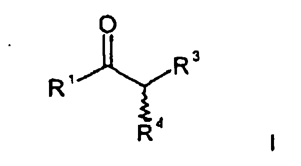 dans lequel un composé de formule II est converti en
un composé de formule I par inversion de la configuration stéréochimique sur l'atome de carbone du groupement α-carbonyle, 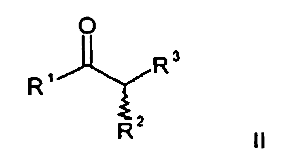 en utilisant des réactifs de types R⁴MgX et ZnY₂ ou R⁴MgX et ZnR⁴₂,
dans lequel R¹ peut représenter un groupement OM, O-R⁵
ou NR⁵R⁶, où M représente un ion de métal et R⁵ et R⁶ peuvent représenter, indépendamment l'un de l'autre, un radical alkyle, un radical alcényle, un radical arylalkyle, un radical aryle ou un radical hétéroaryle, à chaîne linéaire ou ramifiée,
dans lequel R² représente un groupement partant de sulfonate, un groupement partant de phosphate, un groupement partant de carboxylate, un groupement partant de carbamate, un groupement partant de carbonate ou un halogénure,
dans lequel R³ représente un radical alkyle, un radical alcényle, un radical arylalkyle, un radical aryle ou un radical hétéroaryle, à chaîne linéaire ou ramifiée, qui peut présenter 5 à 7 atomes de carbone, ainsi que 1 ou 2 hétéroatomes, choisis parmi les éléments O, N, P et S, comme substituants, ou qui peut être interrompu par les hétéroatomes,
dans lequel R⁴ représente un radical alkyle, un radical alcényle, un radical arylalkyle, un radical aryle ou un radical hétéroaryle, à chaîne linéaire ou ramifiée, dans lequel, dans les radicaux R¹ à R⁶, un radical alkyle peut présenter 1 à 15 atomes de carbone, un radical alcényle peut présenter 2 à 15 atomes de carbone, un radical arylalkyle peut présenter 5 à 15 atomes de carbone, un radical aryle peut présenter 5 à 10 atomes de carbone et un radical hétéroaryle peut présenter 5 à 8 atomes de carbone, ainsi que 1 à 2 hétéroatomes choisi(s) parmi les éléments O, N, P et S,
dans lequel X représente un halogène, et
dans lequel Y peut représenter un sulfonate, un sulfate, un carboxylate, un alcoolate, un nitrate, un chalcogène ou un halogène.

2. Procédé selon la revendication 1, **caractérisé en ce que** le procédé est réalisé à des températures supérieures à -30 °C.

3. Procédé selon l'une quelconque des revendications précédentes, **caractérisé en ce que** l'on utilise des composés de formule II présentant un excès d'énatiomère (ee) supérieur à 95 % et **en ce que** l'on fabrique des composés de formule I présentant un excès d'énantiomère supérieur à 95 %.

4. Procédé selon l'une quelconque des revendications précédentes, **caractérisé en ce que** l'on utilise 0,5 à 25 % en poids de composé ZnY₂ ou ZnR⁴₂ par rapport à la quantité de composé de formule II.

5. Procédé selon l'une quelconque des revendications précédentes, **caractérisé en ce que** l'on utilise, comme solvant pour la réaction, du tétrahydrofuranne, du 2-méthyltétrahydrofuranne ou du diéthyléther ou un mélange de ces solvants.

6. Procédé selon l'une quelconque des revendications précédentes, **caractérisé en ce qu'**un radical alkyle comprend 1 à 10 atomes de carbone, un radical alcényle comprend 2 à 10 atomes de carbone, un radical arylalkyle comprend 5 à 10 atomes de carbone, un radical aryle comprend 5 à 8 atomes de carbone et un radical hétéroaryle comprend 5 à 7 atomes de carbone, ainsi que 1 à 2 hétéroatomes choisis parmi les éléments O, N, P et S.

7. Procédé selon l'une quelconque des revendications précédentes, **caractérisé en ce que** le radical R² représente un groupement acide alkylsulfonique, un groupement phosphate, un groupement carboxylate, un groupement carbamate, un groupement carbonate ou un halogène.

8. Procédé selon la revendication 7, **caractérisé en ce que** le radical R² représente un groupement partant de triflate ou de nonaflate.

9. Procédé selon l'une quelconque des revendications 1 à 5, **caractérisé en ce que** le radical R⁴ représente un groupement méthyle, éthyle, i-propyle, n-butyle, i-butyle, s-butyle, cyclohexyle, octyle, lauryle, allyle, méthallyle, vinyle, phényle, benzyle, 3-benzyloxypropyle et 3-benzyloxy-2-méthylpropyle.

10. Procédé selon l'une quelconque des revendications précédentes, **caractérisé en ce que** l'on utilise 1 à 2 équivalents de réactif R⁴MgX par rapport à la quantité du composé de formule II.

11. Procédé selon l'une quelconque des revendications précédentes, **caractérisé en ce que** les composés de formule II sont fabriqués en partant de composés chiraux naturels.

12. Procédé selon l'une quelconque des revendications précédentes, **caractérisé en ce que** les composés de formule II sont fabriqués à partir de composés choisis dans le groupe constitué des acides aminés, de l'acide L-lactique, de l'acide L-malique, de l'acide L-tartrique et de l'acide L-mandélique.
